# EUROPEAN PATENT SPECIFICATION

(11) **EP 4 137 213 B1**
(45) Date of publication and mention of the grant of the patent: **27.08.2025**
(21) Application number: 21799820.2
(22) Date of filing: 03.03.2021
(51) Int. Cl.: A63B 22/06, A63B 69/16, A63B 71/06, A61B 5/389

(54) **CADENCE MEASUREMENT DEVICE AND CADENCE MEASUREMENT METHOD**
KADENZMESSVORRICHTUNG UND KADENZMESSVERFAHREN
DISPOSITIF DE MESURE DE CADENCE ET PROCÉDÉ DE MESURE DE CADENCE

(30) Priority: 08.05.2020 JP 2020082718
(43) Date of publication of application: 22.02.2023
(73) Proprietor: Alps Alpine Co., Ltd., Tokyo 145-8501 (JP)
(72) Inventor: ITO, Takayuki, Tokyo 145-8501 (JP); ITOH, Jouichi, Tokyo 145-8501 (JP)
(74) Representative: Schmitt-Nilson Schraud Waibel Wohlfrom Patentanwälte Partnerschaft mbB
(86) International application number: PCT/JP2021/008180
(87) International publication number: WO 2021/225025

(56) References cited:
- JP-A- 2007 268 016
- JP-A- 2017 517 715
- JP-A- 2018 110 730
- US-A1- 2019 073 605
- RICHER ROBERT ET AL: "Real-Time ECG and EMG Analysis for Biking Using Android-Based Mobile Devices", 2014 11TH INTERNATIONAL CONFERENCE ON WEARABLE AND IMPLANTABLE BODY SENSOR NETWORKS, IEEE, 16 June 2014 (2014-06-16), pages 104 - 108, XP032620715, DOI: 10.1109/BSN.2014.20
- SATO TAKUHIRO ET AL: "Motor control mechanism underlying pedaling skills: an analysis of bilateral coordination in the lower extremities", ARTIFICIAL LIFE AND ROBOTICS, SPRINGER JAPAN, TOKYO, vol. 25, no. 2, 29 November 2019 (2019-11-29), pages 308 - 315, XP037108955, ISSN: 1433-5298, [retrieved on 20191129], DOI: 10.1007/S10015-019-00580-8
- INOUE TSUYOSHI ET AL: "Real-time Muscle Activity Indication Method for Pedaling Motion using Surface EMG", 2018 IEEE INTERNATIONAL CONFERENCE ON ARTIFICIAL INTELLIGENCE IN ENGINEERING AND TECHNOLOGY (IICAIET), IEEE, 8 November 2018 (2018-11-08), pages 1 - 4, XP033516817, DOI: 10.1109/IICAIET.2018.8638461
- TERASHIMA, SHINCHIRO; INOUE, TSUYOSHI: "EMG Signal Processing Method for Real- time Muscle Activity Indication in Pedaling Motion", 46TH SICE SYMPOSIUM ON INTELLIGENT SYSTEMS; MARCH 6-7, 2019, vol. 46, 6 March 2019 (2019-03-06), JP, pages 1 - 4, XP009541190

## Description

### Technical Field

The present invention relates to a cadence measurement device and a cadence measurement method.

### Background Art

There has been a conventional bicycle-type exercise instrument in which a pedaling measurement unit attached to the bicycle-type exercise instrument has a rotational speed sensor and that measures cadence, which is the rotational speed of a crank portion, according to an output signal of the rotational speed sensor (see PTL 1, for example).

### Citation List

### Patent Literature

PTL 1: Japanese Unexamined Patent Application Publication No. 2018-110730
Furthermore, in the publication "Real-Time ECG and EMG Analysis for Biking Using Android-Based Mobile Devices", by Robert Richer et al., 2014 11TH INTERNATIONAL CONFERENCE ON WEARABLE AND IMPLANTABLE BODY SENSOR NETWORKS, IEEE, 16 June 2014 (2014-06-16), pages 104-108, XP032620715, DOI: 10.1109/BSN.2014.20, an application for Android-based mobile devices is described that enables a real-time calculation of heart rate and cadence for biking. Therefore, both ECG and EMG data are acquired in real time by Shimmer^{™} sensors and transmitted via Bluetooth, as well as processed and evaluated on the mobile device. The ECG algorithm is based on the Pan-Tompkins algorithm for QRS-Detection and offers a heart beat detection rate of more than 94%. The EMG algorithm offers a treadle detection rate of more than 91%. The application's range of features is complemented by GPS data for the calenlation of speed and location information.

### Summary of Invention

The invention is specified in the independent claims.

### Technical Problem

Incidentally, the conventional bicycle-type exercise instrument uses a rotational speed sensor to measure cadence, so the structure is not simple.

Therefore, an object is to provide a cadence measurement device having a simple structure and to provide a cadence measurement method.

### Solution to Problem

A cadence measurement device in an embodiment of the present disclosure includes a myoelectric potential sensor attached to a leg portion of a living body, a waveform processing unit that performs waveform processing to obtain a myoelectric potential waveform from an output signal of the myoelectric potential sensor, and a cadence deriving unit that derives cadence according to the myoelectric potential waveform obtained by the waveform processing.

### Advantageous Effects of Invention

It is possible to provide a cadence measurement device having a simple structure and to provide a cadence measurement method.

### Brief Description of Drawings

[Fig. 1] Fig. 1 is a drawing indicating the structure of a cadence measurement device 100 in an embodiment.
[Fig. 2] Fig. 2 is a drawing explaining waveform processing executed by a waveform processing unit 122.
[Fig. 3] Fig. 3 is a drawing explaining waveform processing executed by the waveform processing unit 122.
[Fig. 4] Fig. 4 is a drawing explaining waveform processing executed by the waveform processing unit 122.
[Fig. 5] Fig. 5 is a drawing explaining waveform processing executed by the waveform processing unit 122.
[Fig. 6] Fig. 6 is a drawing explaining processing executed by a cadence deriving unit 123.
[Fig. 7] Fig. 7 is a drawing indicating table data that relates cadence and the period of one cycle to each other.
[Fig. 8] Fig. 8 is a flowchart indicating processing executed by the cadence measurement device 100.

### Description of Embodiments

Embodiments to which a cadence measurement device and a cadence measurement method in the present invention are applied will be described below.

### <Embodiments>

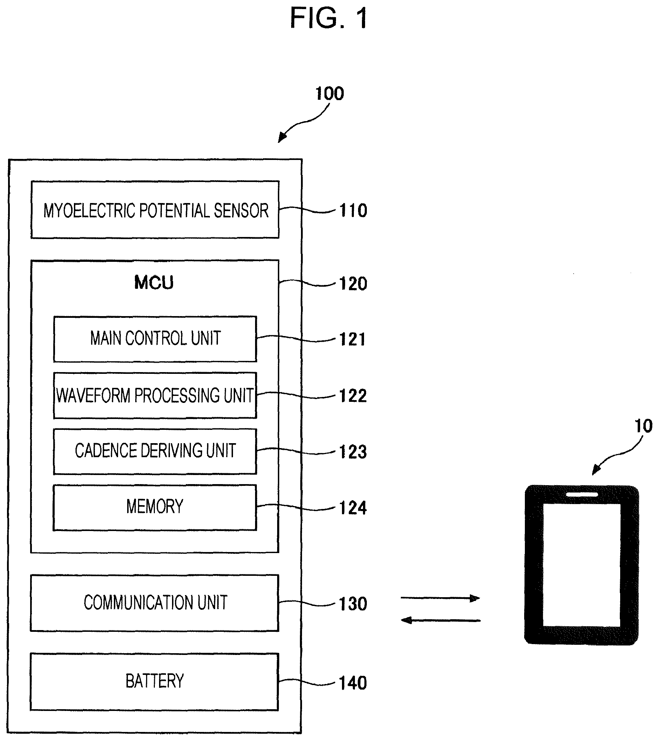

Fig. 1 is a drawing indicating the structure of a cadence measurement device 100 in an embodiment. In Fig. 1, a smart phone 1 is also indicated. In Fig. 1, the cadence measurement device 100 is indicated more largely than the smart phone 10 to indicate the structure of the cadence measurement device 100 in detail. However, the actual cadence measurement device 100 is smaller and more lightweight than the smart phone 10.

The cadence measurement device 100 includes a myoelectric potential sensor 110, a MCU (Micro Control Unit) 120, a communication unit 130, and a battery 140. As an example, the cadence measurement device 100 is attached to the thigh of the user who pedals a bicycle or bicycle-type exercise instrument by being stuck with, for example, a tape or the like. Then, the cadence measurement device 100 measures cadence. Cadence is a number of revolutions of the crank axis of the pedal per minute. The cadence measurement device 100 only needs to measure the myoelectric potential of the surface of the leg portion of the user with the myoelectric potential sensor 110. As an example, the cadence measurement device 100 is stuck so as to measure the myoelectric potential of the quadriceps muscle.

The myoelectric potential sensor 110 has an analog amplifier circuit and a plurality of bioelectrodes stuck to the thigh of the user. The myoelectric potential sensor 110 amplifies a waveform signal, which represents a myoelectric potential detected in a hyperbolic lead method by using the plurality of bioelectrodes, with the analog amplifier circuit, and then outputs the waveform signal. The output of the myoelectric potential sensor 110 is an example of an output signal and is input into the MCU 120.

The MCU 120 has a main control unit 121, a waveform processing unit 122, a cadence deriving unit 123, and a memory 124. The MCU 120 is realized by a microcomputer that includes a CPU (Central Processing Unit), a RAM (Random Access Memory), a ROM (Read Only Memory), and the like. The main control unit 121, waveform processing unit 122, cadence deriving unit 123 indicate the functions of programs executed by the MCU 120 as functional blocks. Also, the memory 124 functionally represents a memory in the MCU 120.

The main control unit 121 is a processing unit that controls the MCU 120 in a centralized manner, and executes processing other than processing executed by the waveform processing unit 122 and cadence deriving unit 123. Specifically, the main control unit 121 performs A/D (Analog to Digital) conversion processing on the output signal of the myoelectric potential sensor 110 to convert the output signal to a digital waveform signal and other processing, for example.

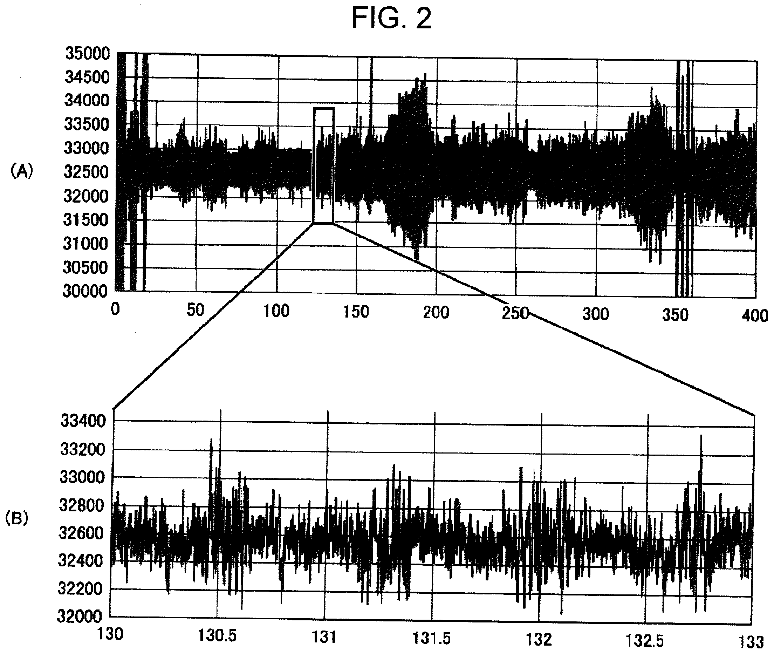

The waveform processing unit 122 acquires data representing a myoelectric potential waveform by performing waveform processing on the output signal of the myoelectric potential sensor 110. Waveform processing performed by the waveform processing unit 122 will be described later by using Fig. 2 to Fig. 5.

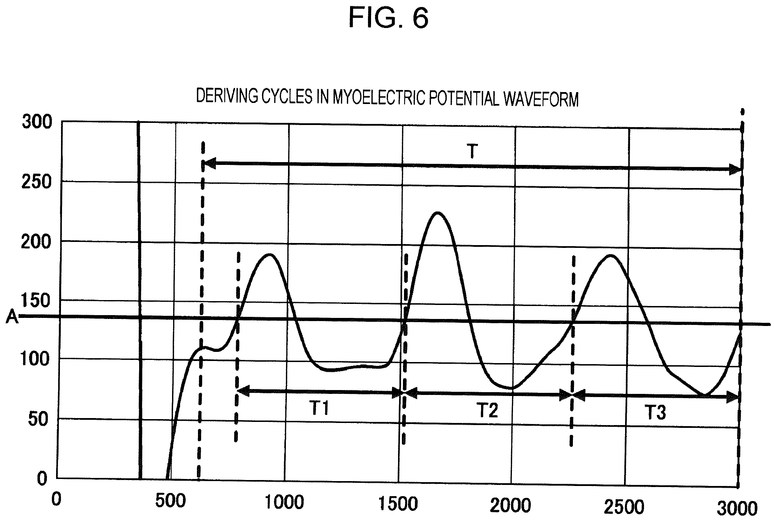

The cadence deriving unit 123 derives cadence from the myoelectric potential waveform acquired by the waveform processing unit 122. Waveform processing performed by the cadence deriving unit 123 will be described later by using Fig.6.

The memory 124 is an example of a storage unit. The memory 124 stores programs and data needed by the MCU 120 to perform processing as well as other data, and also temporarily stores data to be used by the main control unit 121, waveform processing unit 122, and cadence deriving unit 123 in processing and the like.

The communication unit 130 is a communication unit in BLE (Bluetooth Low Energy) (registered trademark) as an example, and performs data communication with the smart phone 10. Measurement results of the cadence measurement device 100 are transmitted to the smart phone 10 through the communication unit 130. Application programs for the cadence measurement device 100 are installed in the smart phone 10, as an example. The smart phone 10 displays or otherwise processes data that an application program has received from the cadence measurement device 100 on the display or the like of the smart phone 10.

The battery 140 is a power source that supplies electric power to the myoelectric potential sensor 110, MCU 120, and communication unit 130. The battery 140 only needs to be a secondary battery, as an example. The battery 140 can be charged in a state in which the battery 140 is incorporated into the cadence measurement device 100 or is removed from the cadence measurement device 100.

Fig. 2 to Fig. 5 are drawings explaining waveform processing executed by the waveform processing unit 122. In Fig. 2 to Fig. 5, the horizontal axis indicates time and the vertical axis indicates amplitude. Fig. 2(A) indicates the waveform of a digital waveform signal created by the main control unit 121 through A/D conversion. Fig. 2(B) indicates part (segment for three seconds) in Fig. 2(A) by enlarging it in the horizontal-axis direction. The digital waveform signal indicated in Figs. 2(A) and (B) represents a myoelectric potential (EMG: ElectroMyoGraphy) but includes noise and the like as well.

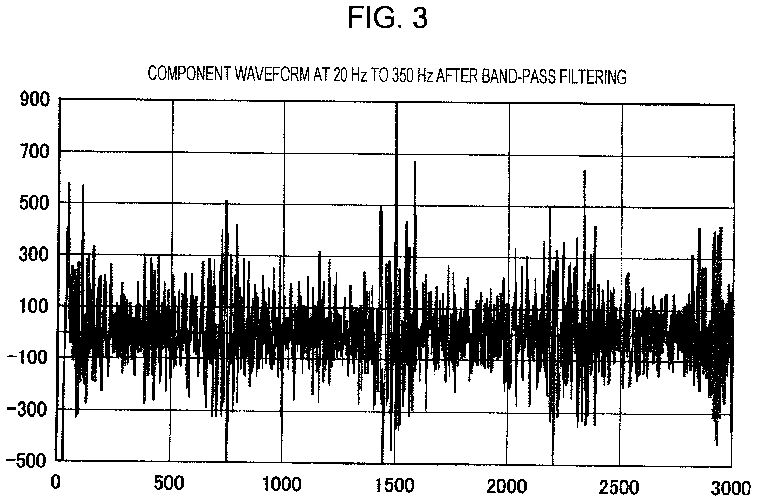

Fig. 3 indicates a component waveform after the waveform processing unit 122 has performed band-pass filter processing on the digital waveform signal indicated in Fig. 2(B). Band-pass filter processing is filter processing (waveform processing) to pass only 20 Hz to 350 Hz, which are in a frequency band that the myoelectric potential waveform can take in the digital waveform signal, and remove components at lower than 20 Hz and components at higher than 350 Hz.

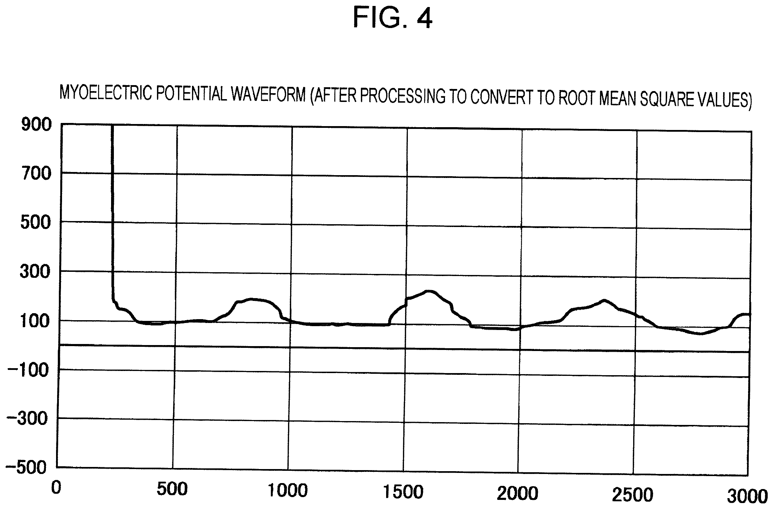

Fig. 4 is a drawing indicating a myoelectric potential waveform obtained when the waveform processing unit 122 performs waveform processing on the component waveform indicated in Fig. 3. By performing waveform processing to convert the amplitude of the component waveform indicated in Fig. 3 to a signal waveform with RMS (Root Mean Square) values, the myoelectric potential waveform indicated in Fig. 4 is obtained. The myoelectric potential waveform is obtained by squaring the positive component and negative component of the component waveform indicated in Fig. 3 and taking a square root.

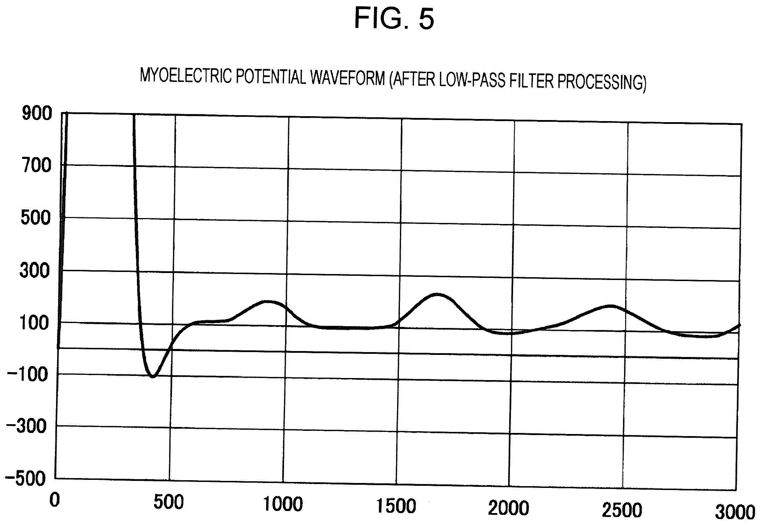

Fig. 5 is a drawing indicating a myoelectric potential waveform that the waveform processing unit 122 obtains by performing low-pass filter processing on the myoelectric potential waveform indicated in Fig. 4. It is said that the number of revolutions per minute when a human pedals a bicycle is 200 rpm (revolutions per minute), which is about 3.35 Hz, or less. Therefore, to extract components at 3.35 Hz or lower from the myoelectric potential waveform indicated in Fig. 4, low-pass filter processing in which 3.35 Hz is used as the cut-off frequency is performed.

Fig. 6 is a drawing explaining processing executed by the cadence deriving unit 123. In Fig. 6, the period T is a three-second period in the myoelectric potential waveform. Since it is thought that the period T includes a plurality of cycles during which the user pedals, the period T is set to three seconds as an example.

The cadence deriving unit 123 obtains the average value A of amplitude data in the period T, and divides the period T into a plurality of cycles with the amplitude center of the myoelectric potential waveform in the period T taken as the average value A. In Fig. 6, three cycles T1, T2, and T3 are included in the period T. There is a residual, which is shorter than one cycle, before cycle T1. The cadence deriving unit 123 obtains periods T1, T2, and T3.

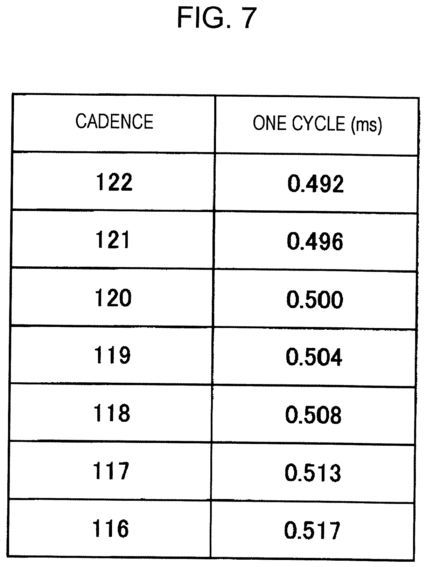

Fig. 7 is drawing indicating table data that relates cadence and the period (ms (milliseconds)) of one cycle to each other. The table data indicated in Fig. 7 is data that relates the period of one revolution (period of one cycle) of cadence used as the number of revolutions of the crank. As an example, Fig. 7 indicates the period (0.517 ms to 0.492 ms) of one cycle when cadence is 115 to 122 (revolutions/minute).

The cadence deriving unit 123 references the table data and derives the cadence for which the period of one cycle is closest to each of periods T1 to T3. As a result, it will be assumed as an example that the cadence deriving unit 123 has derived that cadence in period T1 is 120 (revolutions/minute), cadence in period T2 is 119 (revolutions/minute), and cadence in period T3 is 118 (revolutions/minute). The cadence measurement device 100 obtains cadence from the myoelectric potential waveform in the way described above.

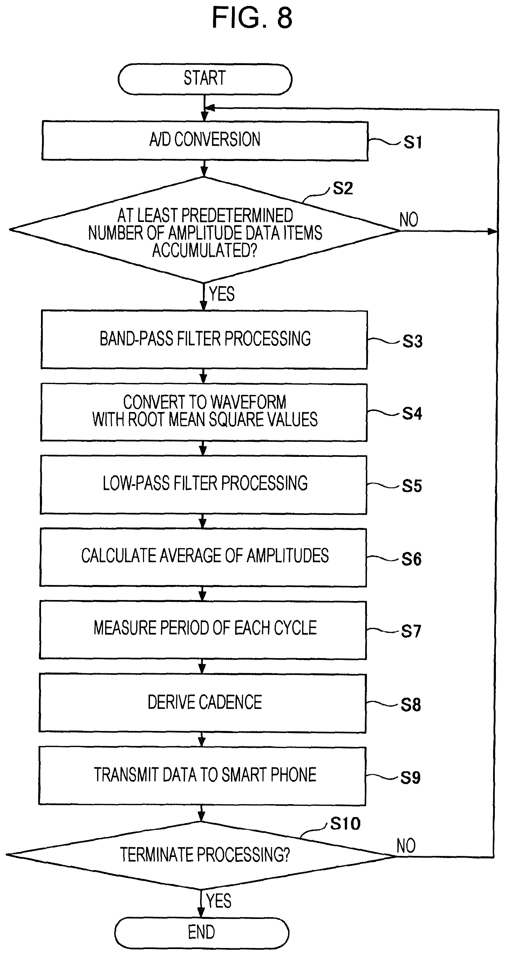

Fig. 8 is a flowchart indicating processing executed by the cadence measurement device 100. As a premise, it will be assumed that the cadence measurement device 100 has been stuck to the thigh of the user, the power supply of the cadence measurement device 100 is turned on, and the myoelectric potential sensor 110 is performing measurement of the myoelectric potential. Processing indicated in Fig. 8 is processing realized by the cadence measurement method in an embodiment.

The main control unit 121 performs processing in which A/D conversion processing is performed on the output signal of the myoelectric potential sensor 110 to convert the output signal to a digital waveform signal (step S1). More specifically, as an example, the main control unit 121 creates a digital waveform signal by performing A/D conversion on the output signal of the myoelectric potential sensor 110 at a sampling cycle of 0.1 ms, and stores amplitude data of the digital waveform signal in the memory 124.

The waveform processing unit 122 decides whether at least a predetermined number of amplitude data items of the digital waveform signal have been accumulated (step S2). Here, at least the predetermined number of amplitude data items of the digital waveform signal refer to amplitude data items of the digital waveform signal for three seconds or more, as an example. This is because the periods of a plurality of cycles included in the period T for three seconds will be obtained later as in Fig. 6.

If the waveform processing unit 122 decides that at least the predetermined number of amplitude data items of the digital waveform signal have been accumulated (S2: YES), the waveform processing unit 122 performs band-pass filter processing on the digital waveform signals (step S3). Due to this, component waveforms after only 20 Hz to 350 Hz, which are in a frequency band that the myoelectric potential waveform can take, in the digital waveform signal has been passed are obtained as indicated in Fig. 3. Incidentally, if the waveform processing unit 122 decides in step S2 that at least the predetermined number of amplitude data items of the digital waveform signal have not been accumulated (S2: NO), the waveform processing unit 122 returns the flow to step S1. This is because the waveform processing unit 122 waits for amplitude data to be further accumulated.

The waveform processing unit 122 performs waveform processing to convert the amplitude of the component waveform to a signal waveform with root mean square (RMS) values (step S4). Due to this, a myoelectric potential waveform as indicated in Fig. 4 is obtained.

The waveform processing unit 122 performs low-pass filter processing on the myoelectric potential waveform (step S5). Due to this, out of the myoelectric potential waveforms obtained in step S4, components at 3.35 Hz or lower are extracted and a myoelectric potential waveform as indicated in Fig. 5 is obtained.

The cadence deriving unit 123 calculates the average value A of amplitude data in the period T (step S6). The period T (see Fig. 6) is a period that includes at least the predetermined number of amplitude data items of the digital waveform signal, which have been obtained in step S2. That is, the period T is determined in step S2.

The cadence deriving unit 123 measures the period of each cycle included in the period T (step S7). As an example, processing in step S7 is processing to measure a plurality of periods T1, T2, and T3 included in the period T, as described by using Fig. 6. Measurement of periods T1, T2, and T3 only needs to be performed by obtaining an interval between sampling points, at which the amplitude data of the myoelectric potential waveform becomes the same as the average value A, in the direction of time. In this case, if there is no sampling point, at which a match with the amplitude data of the average value A is found, in the myoelectric potential waveform, interpolation or the like only needs to be performed for two sampling points between which the average value A is interposed.

The cadence deriving unit 123 references the table data and derives the cadence for which the period of one cycle is closest to each of periods T1 to T3 (step S8).

The main control unit 111 causes the communication unit 130 to transmit data representing cadence to the smart phone (step S9). Due to this, the smart phone 10 acquires the data representing cadence and displays the data on the display. The user can know the cadence by seeing the display of the smart phone 10.

The main control unit 111 decides whether to terminate the processing (step S10). The time to terminate the processing is when a manipulation to terminate measurement processing by the cadence measurement device 100 is performed. If the main control unit 111 decides that the processing is not to be terminated (S10: NO), the main control unit 111 returns the flow to step S1. Also, if the main control unit 111 decides that the processing is to be terminated (S10: YES), the main control unit 111 terminates a series of processing.

As described above, the cadence measurement device 100 obtains a myoelectric potential waveform by performing filter processing on an output signal representing the myoelectric potential detected by the myoelectric potential sensor 110 and performing waveform processing such as processing to convert to root mean square values, and derives cadence from cycles included in the myoelectric potential waveform. In this case, there is no need to use a rotational speed sensor and there is also no need to use an acceleration sensor and the like, as in the past. Due to this, the cadence measurement device 100 can be realized with a very simple structure.

Therefore, it is possible to provide the cadence measurement device 100 having a simple structure and the cadence measurement method. Since the cadence measurement device 100 can be realized only with the myoelectric potential sensor 110, MCU 120, communication unit 130, and battery 140, the cadence measurement device 100 is very small and lightweight. Due to this, even when the user sticks the cadence measurement device 100 to the thigh with a tape or the like, the cadence measurement device 100 is less likely to drop off and cadence measurement can be stably continued for a long period of time. Also, even when the cadence measurement device 100 is stuck to the thigh of the user, there is very little feeling of discomfort, so the cadence measurement device 100 is superior in ease of attachment.

Also, in processing performed by an application program in the smart phone 10 after the myoelectric potential waveform measured by the cadence measurement device 100 is transmitted to the smart phone 10, the degree of fatigue of muscles of the user and the like may be inferred and may be displayed on the display together with cadence. Then, the user can quantitatively grasp the degree of fatigue of muscles of the user together with the cadence.

Incidentally, an aspect has been described above in which the cadence deriving unit 123 measures the period of each cycle included in the period T and derives the cadence for which the period of one cycle is closest to each of periods T1 to T3. However, table data that relates cadence and the number of revolutions of the crank per second to each other may have been stored in the memory 124 instead of the table data indicated in Fig. 7. Then, in the table data, the cadence closest to the reciprocal (number of revolutions of the crank per second) of the period of each cycle may be derived, the reciprocal being obtained by performing a Fourier transform on the myoelectric potential waveform indicated in Fig. 5.

So far, the cadence measurement device and cadence measurement method in exemplary embodiments in the present invention have been described. However, the present invention is not limited to specifically disclosed embodiments. Various variations and modifications are possible without departing from the scope of the claims.

Incidentally, this international application claims priority based on Japanese Patent Application No. 2020-082718 filed on May 8, 2020.

### Reference Signs List

- 100: cadence measurement device
- 110: myoelectric potential sensor
- 120: MCU
- 122: waveform processing unit
- 123: cadence deriving unit

## Claims

1. A cadence measurement device (100), comprising:
a myoelectric potential sensor (110) adapted to be attached to a leg portion of a living body;
a waveform processing unit (122) configured to perform waveform processing to obtain a myoelectric potential waveform from an output signal of the myoelectric potential sensor; and
a cadence deriving unit (123) configured to derive, when an average value of an amplitude in a plurality of cycles of the myoelectric potential waveform obtained by the waveform processing is taken as an amplitude center and the myoelectric potential waveform is divided into each cycle according to the amplitude center, the cadence according to a period of each cycle;
wherein, if the waveform processing unit (122) decides that 3 seconds or more of amplitude data items of a digital waveform signal, obtained by Analog to Digital converting the output signal, have been accumulated, the waveform processing unit (122) is configured to perform band-pass filter processing on the digital waveform signal, wherein the waveform processing unit (122) is configured to extract components at 3.35 Hz or less from the myoelectric potential waveform, wherein the waveform processing unit (122) is configured to obtain the myoelectric potential waveform from the output signal by performing, as the waveform processing, processing to convert the output signal of the myoelectric potential sensor (110) to a signal waveform with a root mean square value, wherein the waveform processing unit (122) is configured to obtain the myoelectric potential waveform from the output signal by performing, as the waveform processing, processing to extract a component waveform, which is a waveform of a frequency component included in the myoelectric potential waveform of the living body, from the output signal of the myoelectric potential sensor (110) and processing to convert the component waveform to a signal waveform with a root mean square value,
wherein the cadence measurement device (100) is configured to perform waveform processing in the following order: S3 band-pass filter processing, S4 convert to waveform with root mean square values, S5 low-pass filter processing, S6 calculate average of amplitudes, S7 measure period of each cycle and S8 derive cadence.

2. The cadence measurement device (100) according to Claim 1, further comprising a storage unit (124) configured to store table data that relates a number of crank revolutions and a period of one cycle, wherein
the cadence deriving unit (123) is configured to derive, as the cadence, the number of crank revolutions that corresponds, in the table data, to the period of each cycle.

3. A microcomputer-implemented cadence measurement method comprising:
performing waveform processing to obtain a myoelectric potential waveform from an output signal of a myoelectric potential sensor (110) attached to a leg portion of a living body; and
deriving, when an average value of an amplitude in a plurality of cycles of the myoelectric potential waveform obtained by the waveform processing is taken as an amplitude center and the myoelectric potential waveform is divided into each cycle according to the amplitude center, cadence according to a period of each cycle;
if it is decided that 3 seconds or more of amplitude data items of a digital waveform signal, obtained by Analog to Digital
converting the output signal, have been accumulated, performing band-pass filter processing on the digital waveform signal, the cadence measurement method, further comprising:
extracting components at 3.35 Hz or less from the myoelectric potential waveform,
the waveform processing unit (122) obtaining the myoelectric potential waveform from the output signal by performing, as the waveform processing, processing to convert the output signal of the myoelectric potential sensor (110) to a signal waveform with a root mean square value,
the waveform processing unit (122) obtaining the myoelectric potential waveform from the output signal by performing, as the waveform processing, processing to extract a component waveform, which is a waveform of a frequency component included in the myoelectric potential waveform of the living body, from the output signal of the myoelectric potential sensor (110) and processing to convert the component waveform to a signal waveform with a root mean square value,
wherein the cadence measurement method processes waveforms in the following order: S3 band-pass filter processing, S4 convert to waveform with root mean square values, S5 low-pass filter processing, S6 calculate average of amplitudes, S7 measure period of each cycle and S8 derive cadence.

## Patentansprüche

1. Trrittfrequenzmessvorrichtung (100), aufweisend:
einen myoelektrischen Potentialsensor (110), der an einem Beinbereich eines lebenden Körpers anbringbar ist;
eine Wellenformverarbeitungseinheit (122), die zum Ausführen einer Wellenformverarbeitung ausgebildet ist, um aus einem Ausgangssignal des myoelektrischen Potentialsensors eine myoelektrische Potentialwellenform zu erhalten; und
eine Trittfrequenz-Ableitungseinheit (123), die dazu ausgebildet ist, wenn ein Durchschnittswert einer Amplitude in einer Mehrzahl von Zyklen der durch die Wellenformbearbeitung erhaltenen myoelektrischen Potentialwellenform als Amplitudenzentrum verwendet wird und die myoelektrische Potentialwellenform in jedem Zyklus entsprechend dem Amplitudenzentrum unterteilt wird, die Trittfrequenz entsprechend einer Periode des jeweiligen Zyklus abzuleiten;
wobei dann, wenn die Wellenformverarbeitungseinheit (122) die Feststellung trifft, dass 3 Sekunden oder mehr von Amplitudendaten eines digitalen Wellenformsignals aufgelaufen sind, die durch Analog-in-Digital-Umwandlung des Ausgangssignals erhalten wurden, die Wellenformverarbeitungseinheit (122) dazu ausgebildet ist, eine Bandpass-Filterverarbeitung an dem digitalen Wellenformsignal auszuführen, wobei die Wellenformverarbeitungseinheit (122) dazu ausgebildet ist, Komponenten bei 3,35 Hz oder weniger aus der myoelektrischen Potentialwellenform zu extrahieren, wobei die Wellenformverarbeitungseinheit (122) dazu ausgebildet ist, die myoelektrische Potentialwellenform aus dem Ausgangssignal zu erhalten, indem sie als Wellenformverarbeitung eine Verarbeitung zum Umwandeln des Ausgangssignals des myoelektrischen Potentialsensors (110) in eine Signalwellenform mit einem quadratischen Mittelwert ausführt, wobei die Wellenformverarbeitungseinheit (122) dazu ausgebildet ist, die myoelektrische Potentialwellenform aus dem Ausgangssignal zu erhalten, indem sie als Wellenformverarbeitung eine Verarbeitung zum Extrahieren einer Komponentenwellenform, die eine Wellenform einer in der myoelektrischen Potentialwellenform des lebenden Körpers enthaltenen Frequenzkomponente ist, aus dem Ausgangssignal des myoelektrischen Potentialsensors (110) ausführt sowie eine Verarbeitung zum Umwandeln der Komponentenwellenform in eine Signalwellenform mit einem quadratischen Mittelwert ausführt,
wobei die Trittfrequenzmessvorrichtung (100) dazu ausgebildet ist, die Wellenformverarbeitung in der folgenden Reihenfolge auszuführen: S3 Bandpass-Filterverarbeitung, S4 Umwandeln in Wellenform mit quadratischen Mittelwerten, S5 Tiefpass-Filterverarbeitung, S6 Berechnung des Amplitudendurchschnitts, S7 Messung der Periode jedes Zyklus und S8 Ableiten der Trittfrequenz.

2. Trittfrequenzmessvorrichtung (100) nach Anspruch 1,
die ferner eine Speichereinheit (124) aufweist, die zum Speichern von Tabellendaten ausgebildet ist, die sich auf eine Anzahl von Kurbelumdrehungen und eine Periode eines Zyklus beziehen, wobei die Trittfrequenzableitungseinheit (123) dazu ausgebildet ist, als Trittfrequenz die Anzahl von Kurbelumdrehungen abzuleiten, die in den Tabellendaten der Periode des jeweiligen Zyklus entspricht.

3. Microcomputer-implementiertes Trittfrequenzmessverfahren, das folgende Schritte aufweist:
Ausführen einer Wellenformverarbeitung, um eine myoelektrische Potentialwellenform aus einem Ausgangssignal eines an einem Beinbereich eines lebenden Körpers angebrachten myoelektrischen Potentialsensors (110) zu erhalten; und
wenn ein Durchschnittswert einer Amplitude in einer Mehrzahl von Zyklen der durch die Wellenformbearbeitung erhaltenen myoelektrischen Potentialwellenform als Amplitudenzentrum verwendet wird und die myoelektrische Potentialwellenform in jedem Zyklus entsprechend dem Amplitudenzentrum unterteilt wird, Ableiten der Trittfrequenz entsprechend einer Periode des jeweiligen Zyklus;
bei Feststellung, dass 3 Sekunden oder mehr von Amplitudendaten eines digitalen Wellenformsignals aufgelaufen sind, die durch Analog-in-Digital-Umwandlung des Ausgangssignals erhalten wurden, Ausführen einer Bandpass-Filterverarbeitung an dem digitalen Wellenformsignal, wobei das Trittfrequenzmessverfahren ferner aufweist:
Extrahieren von Komponenten bei 3,35 Hz oder weniger aus der myoelektrischen Potentialwellenform,
wobei die Wellenformverarbeitungseinheit (122) die myoelektrische Potentialwellenform aus dem Ausgangssignal erhält, indem sie als Wellenformverarbeitung eine Verarbeitung zum Umwandeln des Ausgangssignals des myoelektrischen Potentialsensors (110) in eine Signalwellenform mit einem quadratischen Mittelwert ausführt,
wobei die Wellenformverarbeitungseinheit (122) die myoelektrische Potentialwellenform aus dem Ausgangssignal erhält, indem sie als Wellenformverarbeitung eine Verarbeitung zum Extrahieren einer Komponentenwellenform, die eine Wellenform einer in der myoelektrischen Potentialwellenform des lebenden Körpers enthaltenen Frequenzkomponente ist, aus dem Ausgangssignal des myoelektrischen Potentialsensors (110) ausführt sowie eine Verarbeitung zum Umwandeln der Komponentenwellenform in eine Signalwellenform mit einem quadratischen Mittelwert ausführt,
wobei das Trittfrequenzmessverfahren Wellenformen in der folgenden Reihenfolge verarbeitet: S3 Bandpass-Filterverarbeitung, S4 Umwandeln in Wellenform mit quadratischen Mittelwerten, S5 Tiefpass-Filterverarbeitung, S6 Berechnung des Amplitudendurchschnitts, S7 Messung der Periode jedes Zyklus und S8 Ableiten der Trittfrequenz.

## Revendications

1. Dispositif de mesure de cadence (100) comprenant :
un capteur de potentiel myoélectrique (110) adapté pour être fixé à une partie de jambe d'un corps vivant ;
une unité de traitement de formes d'ondes (122) configurée de manière à effectuer un traitement de formes d'ondes afin d'obtenir une forme d'onde de potentiel myoélectrique à partir d'un signal de sortie du capteur de potentiel myoélectrique ; et
une unité de détermination de la cadence (123) configurée de manière à déterminer la cadence selon une période de chaque cycle, lorsqu'une valeur d'amplitude moyenne sur une pluralité de cycles de la forme d'onde du potentiel myoélectrique obtenue par le traitement de formes d'ondes est prise comme centre d'amplitude et que la forme d'onde du potentiel myoélectrique est divisée entre chaque cycle en fonction du centre d'amplitude ;
dans lequel, si l'unité de traitement de formes d'ondes (122) décide que trois secondes ou plus d'éléments de données d'amplitude d'un signal de forme d'onde numérique, obtenu par conversion analogique-numérique du signal de sortie, ont été accumulées, l'unité de traitement de formes d'ondes (122) est configurée de manière à effectuer un traitement par filtre passe-bande sur le signal de forme d'onde numérique, dans lequel l'unité de traitement de formes d'ondes (122) est configurée de manière à extraire des éléments à 3,35 Hz ou moins de la forme d'onde du potentiel myoélectrique, dans lequel l'unité de traitement de formes d'ondes (122) est configurée de manière à obtenir la forme d'onde du potentiel myoélectrique à partir du signal de sortie en effectuant, en tant que traitement de forme d'onde, un traitement
visant à convertir le signal de sortie du capteur de potentiel myoélectrique (110) en forme d'onde de signal avec une valeur quadratique moyenne, dans lequel l'unité de traitement de formes d'ondes (122) est configurée de manière à obtenir la forme d'onde du potentiel myoélectrique à partir du signal de sortie en effectuant, en tant que traitement de forme d'onde, un traitement visant à extraire une forme d'onde d'élément, qui est une forme d'onde d'un élément de fréquence inclus dans la forme d'onde du potentiel myoélectrique du corps vivant, à partir du signal de sortie du capteur de potentiel myoélectrique (110) et un traitement visant à convertir la forme d'onde de l'élément en une forme d'onde de signal avec une valeur quadratique moyenne,
dans lequel le dispositif de mesure de cadence (100) est configuré de manière à effectuer le traitement de la forme d'onde dans l'ordre suivant : S3 traitement par filtre passe-bande, S4 conversion en forme d'onde avec des valeurs quadratiques moyennes, S5 traitement par filtre passe-bas, S6 calcul d'une moyenne d'amplitudes, S7 mesure d'une période de chaque cycle et S8 détermination de la cadence.

2. Le dispositif de mesure de cadence (100) selon la revendication 1, comprenant en outre une unité de stockage (124) configurée de manière à stocker des données de tableau qui mettent en relation un nombre de tours de manivelle et une période d'un cycle, dans lequel
l'unité de détermination de la cadence (123) est configurée de manière à déterminer, en tant que cadence, le nombre de tours de manivelle qui correspond, dans les données du tableau, à la période de chaque cycle.

3. Procédé de mesure de cadence mis en œuvre par un micro-ordinateur consistant à :
effectuer un traitement de forme d'onde pour obtenir une forme d'onde de potentiel myoélectrique à partir d'un signal de sortie d'un capteur de potentiel myoélectrique (110) fixé à une partie de jambe d'un corps vivant ; et
déterminer la cadence selon une période de chaque cycle, lorsqu'une valeur d'amplitude moyenne sur une pluralité de cycles de la forme d'onde du potentiel myoélectrique obtenue par le traitement de formes d'ondes est prise comme centre d'amplitude et que la forme d'onde du potentiel myoélectrique est divisée entre chaque cycle en fonction du centre d'amplitude ;
s'il est décidé que trois secondes ou plus d'éléments de données d'amplitude d'un signal de forme d'onde numérique, obtenu par conversion analogique-numérique du signal de sortie, ont été accumulées, effectuer un traitement par filtre passe-bande sur le signal de forme d'onde numérique, le procédé de mesure de cadence consistant en outre à :
extraire des éléments à 3,35 Hz ou moins de la forme d'onde du potentiel myoélectrique,
l'unité de traitement de formes d'ondes (122) obtenant la forme d'onde du potentiel myoélectrique à partir du signal de sortie en effectuant, en tant que traitement de forme d'onde, un traitement visant à convertir le signal de sortie du capteur de potentiel myoélectrique (110) en forme d'onde de signal avec une valeur quadratique moyenne,
l'unité de traitement de formes d'ondes (122) obtenant la forme d'onde du potentiel myoélectrique à partir du signal de sortie en effectuant, en tant que traitement de forme d'onde, un traitement visant à extraire une forme d'onde d'élément, qui est une forme d'onde d'un élément de fréquence inclus dans la forme d'onde du potentiel myoélectrique du corps vivant, à partir du signal de sortie du capteur de potentiel myoélectrique (110) et un traitement visant à convertir la forme d'onde de l'élément en une forme d'onde de signal avec une valeur quadratique moyenne,
dans lequel le procédé de mesure de cadence traite les formes d'ondes dans l'ordre suivant : S3 traitement par filtre passe-bande, S4 conversion en forme d'onde avec des valeurs quadratiques moyennes, S5 traitement par filtre passe-bas, S6 calcul d'une moyenne d'amplitudes, S7 mesure d'une période de chaque cycle et S8 détermination de la cadence.
